# EUROPÄISCHE PATENTSCHRIFT

(11) **EP 0 938 279 B1**
(45) Veröffentlichungstag und Bekanntmachung des Hinweises auf die Patenterteilung: **31.03.2004**
(21) Anmeldenummer: 98938572.9
(22) Anmeldetag: 24.08.1998
(51) Int. Cl.: A61F 2/30, A61L 27/00, A61F 2/32

(54) **PROTHESE AUS KERAMISCHEN WERKSTOFFEN**
PROSTHESIS MADE FROM CERAMIC MATERIALS
PROTHESE EN MATIERE CERAMIQUE

(30) Priorität: 08.09.1997 CH 209697
(43) Veröffentlichungstag der Anmeldung: 01.09.1999
(73) Patentinhaber: METOXIT AG, CH-8240 Thayngen (CH)
(72) Erfinder: RIEGER, Wolfhart, CH-8263 Buch (CH); WOLF, Hans, CH-8240 Thayngen (CH); FLUCK, Herbert, D-78176 Blumberg (DE)
(74) Vertreter: Peege, Klaus
(86) Internationale Anmeldenummer: PCT/CH1998/000362
(87) Internationale Veröffentlichungsnummer: WO 1999/012497

(56) Entgegenhaltungen:
- EP-A- 0 254 470
- EP-A- 0 707 838
- WO-A-93/23204
- WO-A-97/31592
- US-A- 4 535 486
- US-A- 4 964 869
- US-A- 5 092 898
- US-A- 5 549 697
- US-A- 5 594 651

## Beschreibung

Die Erfindung betrifft ein endoprothetisches Gelenk aus keramischen Werkstoffen, insbesondere Endoprothesen nach dem Oberbegriff des Patentanspruches 1.

Ein derartiges Gelenk ist aus dem Dokument WO-A- 97/31592 bekannt.

Unter Endoprothesen werden Kunstkörper verstanden, die vermittels eines chirurgischen Eingriffes implantiert, d.h. einem Träger eingesetzt werden, um einen gleichen, gegebenenfalls pathologisch veränderten natürlichen Körperbestandteil zu ersetzen. Von der Vielzahl von Endoprothesen liegt die Erfindung auf dem Gebiet solcher, die zum Ersatz von Teilen des Knochengerüstes oder von Gelenken von Lebewesen, insbesondere menschlichen Lebewesen bestimmt sind. Die Erfindung wird folgend im Zusammenhang mit einer Hüftprothese beschrieben, darauf ist die Erfindung jedoch nicht beschränkt, sie erstreckt sich vielmehr auch auf alle anderen Kunstkörper, die zur Ueberwindung der aus dem Stand der Technik bekannten Nachteile nach der erfindungsgemässen Lehre ausbildbar sind. Unter solche Kunstkörper fallen neben Hüftgelenken beispielsweise auch andere Gelenke, wie Knie-, Finger- und Fussgelenke.

Auf dem Gebiet der Humanmedizin bestimmen sich die an eine Endoprothese zu stellenden Gütekriterien nach der Gebrauchsdauer, ergänzt um solche chemischer /medizinischer und technisch/mechanischer Art, die gesamthaft nach Implantation den sogenannten postoperativen Patienten-Komfort (folgend kurz Komfort genannt) einer Endoprothese bestimmen. Unter solche Ergänzungen fallen die Verträglichkeit von Prothesenmaterialien mit Gewebe und Knochen und Körperflüssigkeit (Elektrolyt) eines Emfängers, d.h. die Bio-Kompatibilität von Prothesenmaterialien, Auswirkungen des Abriebes von Gelenkprothesen, die mechanische Festigkeit belasteter Prothesenteile, ihre Tribologie, Wärmeentwicklungen unter Reibung etc.

Für Hüftgelenksprothesen (folgend kurz Hüftprothesen) bestehend aus einem metallischen Schaft mit daran angeordneter Kugel und einer separaten Kalotte, in der sich die Kugel bewegt, sind die verschiedensten Werkstoffkombinationen für Kugel und Kalotte bekannt.

Die bekannteste und am meisten verbreitete Kombination ist die Paarung Metall (Kugel) Polyethylen (Kalotte). Problematisch bei Prothesen dieser Ausgestaltung ist der Metall- und der Polyethylenabrieb. Der Metallabrieb kann Erkrankungen, beispielsweise die Metallose auslösen, während der Polyethylenabrieb (folgend kurz PE-Abrieb) die Verankerung der Prothese im Knochengerüst schwächt. Die Lebensdauer von Prothesen dieser Materialkombination liegt in der Grössenordnung von ca. 10 Jahren, danach ist im Bedarfsfall ein Ersatz zumeist unvermeidlich. In Ansehung des damit verbundenen operativen Aufwandes, kann der Komfort einer solchen Prothese nur als begrenzt eingestuft werden.

Bekannt ist ferner die Materialkombination Aluminiumoxid (Kugel) PE (Kalotte). Mit dieser Kombination ist der Metallabrieb vermieden, nicht vollständig unterdrückt jedoch ist der PE-Abrieb, wobei zum PE-Abrieb ein wenn auch geringes Bruchrisiko für die Kugel hinzutritt. Bei normaler Prothesebelastung wird bei Prothesen dieser Art von einer Lebensdauer von ca. zehn bis 15 Jahren ausgegangen. Eine zehn bis fünfzehnjährige Gebrauchsdauer definiert das heute allgemein akzeptierte, wenn auch nicht als befriedigend taxierte Komfortniveau.

Eine Verbesserung und zwar eine Gebrauchsdauer von zehn bis 20 Jahre bringt die Materialkombination, Metall (Kugel) und Metall (Kalotte). Diese Kombination ist nicht mit einem Bruchrisiko für die Kugel und der Lockerungsproblematik zufolge des PE-Abriebes belastet. Der Metallabrieb ist bei dieser Materialkombination jedoch ein ungelöstes Problem. Der Komfort von Gelenkprothesen dieser Materialkombination wird somit ausserordentlich von der Metallose-Problematik belastet.

Die Suche nach metallfreien Materialkombinationen mit hoher Festigkeit führte zur bekannten Paarung von Zirkonoxid (Kugel) und PE (Kalotte). Die Zielsetzung der Metallfreiheit bei hoher Festigkeit und geringem Bruchrisiko wird mit dieser Paarung erreicht, ungelöst bleibt die Lockerungsgefahr durch den PE-Abrieb. Die Lebensdauer dieses Gelenkes liegt in der Grössenordnung von zehn bis zwanzig Jahren, dieser gehobene Komfort wird jedoch von Lockerungsproblemen belastet.

Zusätzlich zu den Keramik-Weichpaarungen (Al₂O₃ gegen PE, ZrO₂ gegen PE) ist auch eine Keramik-Hartpaarung Aluminiumoxid (Kugel) gegen Aluminiumoxid (Kalotte), also eine Paarung zweier keramischer Materialien, bekannt geworden. Letzere Hartpaarung vereint auf sich sehr gute tribolosche Eigenschaften und Meidung der aus dem Stand der Technik bekannten Abriebsprobleme. Diese Materialpaarung würde ein entsprechend ausgebildetes Gelenk, insbesondere Hüftgelenk, auf ein befriedigendes Komfortniveau (grösser 20 Jahre gesicherte Gebrauchsdauer) heben, wenn die Bruchanfälligkeit der Endoprothese als System (Kugel, Kalotte) eliminiert, zumindest jedoch deutlich reduziert wäre.

Hiervon ausgehend hat sich der Erfinder die Aufgabe gestellt, eine Endoprothese, insbesondere eine Gelenk-Endoprothese aus einer keramischen Hartpaarung zu schaffen, die die Nachteile der bekannten Endoprothesen meidet, und die Aufgabe wird durch eine Endoprothese mit den kennzeichnenden Merkmalen des Patentanspruches 1 gelöst.

Die Verwendung von Zirkonoxid ist auf dem Gebiet der Prothetik, insbesondere auf dem Gebiet der Gelenk-Endoprothesen auf ZrO₂ Weichpaarungen (ZrO₂ gegen PE) beschränkt geblieben. In Hartpaarungen hat Zirkonoxid keinen Eingang gefunden, obwohl die hohe Festigkeit von Zirkonoxid bekannt ist. Der steht nämlich nach Implantation eine Verschleissanfälligkeit der Zirkonoxid-Endoprothesen oder Teilen davon in wässrigen Elektrolyten, z.B. Körperelektrolyten gegenüber, die deutlich höher ist als diejenige anderer keramischer Materialien, z.B. Aluminiumoxid in gleicher Umgebung.

Trotz der bekannten guten Eigenschaften, wie hohe Bruchfestigkeit, ist es diese unter Implantationsbedingungen zu beobachtende vergleichsweise tiefe Verschleissfestigkeit, die die Fachwelt dazu bestimmte, Zirkonoxid allein oder in Kombination mit anderen keramischen Materialien als Hartpaarungsteilnehmer auszugrenzen. Mit der Erfindung wird zur Fortbildung der bekannten (Al₂O₃ gegen Al₂O₃) Hartpaarungs-Endoprothese diese Ausgrenzung überwunden, indem sie eine Werkstoffpaarung vorschlägt, bei der die Verschleissanfälligkeit des Zirkonoxides unter Implantationsbedingungen überraschenderweise entgegen der herrschenden Lehrmeinung weitgehend eliminiert ist.

Für die überraschende Elimination der Verschleissanfälligkeit, d.h. Schaffung der notwendigen Verschleiss- oder Abriebsfestigkeit bezeichnet der Erfinder die Ableitung der bei Betrieb der Prothese in dem Zirkonoxyd-Teil entstehenden Wärme für massgebend, was gemäss der Erfindung durch einen höherwertig wärmeleitenden Hartpaarungs-Werkstoff als Zirkonoxid (Wärmeleitfähigkeit 1,5 bis 2,5 W/mK) erreicht wird. Zu letzteren gehören keramische Werkstoffe mit einer Wärmeleitfähigkeit von 20 bis 300 Watt pro meter Kelvin (W/mK), beispielsweise Aluminiumoxid, Aluminiumnitrid, Berylliumoxid, Bornitrid, Kohlenstoff, Siliziumkarbid und Siliziumnitrid (gesintert und heissgepresst), deren Wärmeleitfähigkeiten in den angegebenen Bereich fallen. Untersuchungen an Hartpaarungsprothesen (Zirkonoxid gepaart mit Aluminiumoxid als Partner mit einer Wärmeleitfähigkeit des Al₂O₃ von 30 W/mK, also am unteren Ende des angegebenen Bereiches) ergaben überraschenderweise, dass Oberflächen von Protheseteilen aus Zirkonoxid bei Wärmeableitung in genannter Umgebung keine der sonst üblichen Alterungserscheinungen aufweisen, die für die vergleichsweise tiefe Verschleissfestigkeit ursächlich sind, und die zu besagter Ausgrenzung des Zirkonoxides führte. Zwischen Alterung und Verschleissfestigkeit besteht ein direkter Zusammenhang, indem gealterte Zirkonoxid-Oberflächen zu starkem Verschleiss bis hin zum Austritt von Körnern aus Oberflächen einer Prothese führen. Wird die Alterung eliminiert, steigt die Verschleissfestigkeit entsprechend. Alterung einer Zirkonoxid-Oberfläche ist eine zunächst oberflächliche Verarmung an Yttriumoxid (Stabilisator für Zirkonoxid), die bei punktuellen Überhitzungen der Oberfläche zufolge von Druckbelastungen letzterer in Körperelektrolyten auftritt. Mit der Erfindung wird eine neue Keramik-Bipaarungsprothese (ZrO₂/Al₂O₃ als Beispiel) geschaffen, deren Komfort den der bekannten Keramik-Monopaarungsprothese (Al₂O₃/Al₂O₃) übertrifft. Im übrigen war überraschenderweise festzustellen, dass sich die Eigenschaften der Prothese als Ganzes, d.h. als System nicht an den Eigenschaften des konstruktiv und/oder mechanisch/technologisch geringerwertigen Paarungspartners ausrichteteten oder orientierten, sondern sich Werte einstellten, die mindestens deutlich (10 - 50 %) oberhalb der des schwächeren Paarungsteilnehmer liegen. Bei der ZrO₂ / Al₂O₃ Keramik-Bipaarungsprothese versagte das Teil aus Aluminiumoxid durch Bruch (Bruckfestigkeit) bei Belastungen, die deutlich über den einer Al₂O₃ Monopaarungsprothese lagen.

Die Fortbildung der bekannten keramischen Monopaarungsprothese (Al₂O₃ gegen Al₂O₃) beruht auf
(i) der Überwindung der Ausgrenzung von Zirkonoxid als Partner einer keramischen Bipaarungsprothese (ZrO₂ gegen ein anorganisches, d.h. keramisches Material mit guter Wärmeleitfähigkeit), und
(ii) dem überraschenden Ergebnis, dass konstruktiv und/oder mechanisch / technologische Eigenschaften (z.B. mechanische Belastbarkeit) des schwächeren Paarungsteilnehmers nicht massgebend sind für die Eigenschaften einer Bipaarungsprothese als System. Dies wird mit folgenden Vergleichsversuchen belegt.

Versuchsobjekte: Zwei Hüftendprothese aus Hartkeramik und zwar eine Monopaarungsprothese (Al₂O₃ gegen Al₂O₃) und eine Bipaarungsprothese (ZrO₂ gegen Al₂O₃).

### Den Vergleichsprothesen gemeinsam war:

Konstruktiv: Kalotten aus Aluminiumoxid.
Abmessungen: Durchmesser 28,04 bis 28,10 mm, vorzugsweise 28,05 mm, Rundheitsabweichung maximal 1,0 bis 2,2 Mikrometer, vorzugsweise 2,0 Mikrometer, Rauhtiefe der Kalottenoberfläche vor Versuchsdurchführung (RA Anfang) 0,006 bis 0,01 Mikormeter, vorzugsweise 0,008 Mikrometer.

### Kugeln aus Aluminiumoxid, oder aus Zirkonoxid:

Abmessungen: Durchmesser 27,970 bis 27,990 mm, vorzugsweise 27,980 mm, Rundheitsabweichung maximal 0,1 bis 2,0 Mikrometer, vorzugsweise 0,15 Mikrometer. Rauhtiefe der Kugeloberfläche vor Versuchsdurchführung (RA-Anfang) 0,004 bis 0,006 Mikrometer, vorzugsweise 0,005 Mikrometer.
Materialzusammensetzung: Aluminiumoxid, Reinheit 99,9 % Al₂O₃ mit einem Gehalt von 0,05 % MgO, Rest übliche Aluminium-Oxidbegleiter, charakterisiert durch folgende mechanisch/technologische Eigenschaftswerte: biaxiale Festigkeit von 390 bis 410 Mega Pascal (MPa) erfindungsgemäss bevorzugt 400 MPa, Elastizitäsmodul von 420 Giga Pascal (GPa), Vickershärte von 2000 bis 2300 Härte Vickers (HV), erfindungsgemäss bevorzugt 2100 HV, Raumgewicht 3,98 Gramm pro Kubikzentimeter (g/cm³) offene Porosität von Null, mittlere Korngrösse (mean linear intercept) von 1,6 bis 2,1 Mikrometer (µM) erfindungsgemäss bevorzugt 1,8 µM.

### Den Vergleichsporthesen nicht gemeinsam war:

Konstruktiv: Bipaarungsprothese, Kugel aus Zirkonoxid; Monopaarungsprothese, Kugel aus Aluminiumoxid.
Materialzusammensetzung: Aluminiumoxid der Kugel der Monopaarungsprothese war in seiner Zusammensetzung identisch der der Kalotte. Zirkonoxid (ZrO₂) und Hafniumoxid (HfO₂) zwischen 94,1 und 95,0 %, erfindungsgemäss bevorzugt 94,7 %, Yttriumoxid (Y₂O₃) zwischen 4,5 und 5,4 %, erfindungsgemäss bevorzugt 5,1 %, maximal 0,5 % andere Oxide, charakterisiert durch folgende mechanisch/technologische Eigenschaftswerte: biaxiale Festigkeit 750 - 900 Mega Pascal (MPa), erfindungsgemäss bevorzugt 850 MPa, Elastizitätsmodul 210 Giga Pascal (GPA), Vickershärte von 1200 bis 1250 HV, erfindungsgemäss bevorzugt 1220 HV, Raumgewicht 6,08 bis 6,09 Gramm pro Kubikzentimeter (g/cm³), erfindungsgemäss bevorzugt 6,085 g/cm³, offene Porosität von Null, mittlere Korngrösse (mean linear intercept) von 0,35 bis 0,45 Mikrometer (erfindungsgemäss bevorzugt 0,4 µM).

### Vorrichtung zur Durchführung der Vergleichsversuche:

Zur Durchführung der Vergleichsversuche wurde ein Zweiachsentribometer verwendet. Ein Zweiachsentribometer kennzeichnet sich durch eine obere Halterung, die um eine senkrechte Achse (Objektachse) drehbar.und eine untere Halterung, die um eine Achse senkrecht zur Objektachse schwenkbar ist. Die Antriebe der oberen und unteren Halterung sind so ausgestaltet, Teildrehungen und -schwenkungen ausführen zu können. Weiter sind die Halterungen mit Lasten beaufschlagbar und arbeiten in einem Flüssigkeitsbehälter.

Zur Versuchsdurchführung waren die Kalotten in der oberen Halterung, die Kugeln in der unteren Halterung so aufgenommen, dass Kalotten und Kugeln miteinander in Eingriff standen, dieser Eingriff, die tatsächlichen Belastungs- und Eingriffsbedingungen der Kugeln und Kalotten nach Implantation simulierend. Diese Bedingungen sind ergänzt dadurch, dass die Kalotten und Kugeln während der Versuchsabläufe in einer sogenannten Ringer-Lösung mit einer Temperatur von 37 °C aufgenommen wären. Diese Lösung und Temperatur repräsentieren den Körperelektrolyt und die Körpertemperatur in bzw. unter denen Endoprothesen ihre Funktionen vollziehen. Die Kalotten wurden um die senkrechte Achse (Objektachse) um +/- 15° (Winkelgrade), die Kugeln um +/- 15° (Winkelgrade) um die Achse senkrecht zur Objektachse geschwenkt. Die Frequenz der Kalottendrehung (Drehfrequenz) betrug 1 Hz (1 pro Sekunde), die Frequenz der Kugelschwenkung (Schwenkfrequenz) 1,183 Hz, eine Drehung und Schwenkung einen Bewegungszyklus bildend. Mit den unterschiedlichen Frequenzen wird grösstmögliche Überdeckung von Kugel- und Kalottenoberfläche erreicht. Eine Versuchsdauer umfasste immer drei Millionen Zyklen, wobei eine Kalotte während der ersten Million Zyklen mit einer Druckbelastung von 100 kg, während der zweiten Million Zyklen mit einer Druckbelastung von 150 kg und während der dritten Million Zyklen mit einer Druckbelastung von 200 kg belegt war.

### Versuchsergebnisse und Interpretation:

Verschleissfestigkeit: Die Verschleissfestigkeit wurde bestimmt durch Messung des Verschleisses, bestimmt durch die maximale Rauhtiefe zu Anfang eines Versuchsprogrammes (RA max. Anfang) und die maximale Rauhtiefe am Ende eines Versuchsprogrammes (RA max. Ende). Die Messungen wurden jeweils für Kugel und Kalotte einer Monopaarungsprothese (Al₂O₃ / Al₂O₃) und Bipaarungsprothese (Al₂O₃ / ZrO₂) vorgenommen.

Interpretation: Verglichen wurden die Messwerte der Monopaarungsprothese mit denen der Bipaarungsprothese. Gleiche oder vergleichbare Messwerte wurden dahingehend interpretiert, dass die Bipaarungsprothese eine bessere, zumindest aber eine gleiche oder vergleichbare Verschleissfestigkeit wie die Monopaarungsprothese oder umgekehrt aufweist.

### Verschleissmessung:

| Al₂O₃ gegen Al₂O₃ | | |
|---|---|---|
| Kugel: | RA max Anfang | 0,005 Mikron |
| | RA max Ende | 0,250 Mikron |
| Kalotte | RA max Anfang | 0,008 Mikron |
| | RA max Ende | 0,200 Mikron |

| ZrO₂ gegen Al₂O₃ | | |
|---|---|---|
| Kugel: | RA max Anfang | 0,005 Mikron |
| | RA max Ende | 0,130 Mikron |
| Kalotte | RA max Anfang | 0,008 Mikron |
| | RA max Ende | 0,180 Mikron |

### Messung der mechanischen Belastbarkeit:

Die Messung der mechanischen Belastbarkeit der Kugel/Kalotten-Systeme aus ZrO₂/Al₂O₃ und Al₂O₃/Al₂O₃ erfolgte nach konventionellen Methoden, indem die Systeme bis zum Bruch eines Systemteiles mit Kräften steigender Grösse belastet würden. Bei Al₂O₃/Al₂O₃ Paarungen erfolgten Brüche (überwiegend der Kugel) bei Kräften ab 50 KN (Kilo Newton) gelegentlich, mit Sicherheit bei aufgebrachten Kräften von 100 KN. Bei ZrO₂/Al₂O₃ Paarungen erfolgten gelegentlich Brüche (überwiegend der Kalotte) bei Kräften ab 80 KN und mit Sicherheit bei Kräften von 150 KN. Diese Versuche belegen, dass eine Bipaarungsprothese mechanisch höher belastbar ist, als die Belastbarkeit ihres konstruktiv und/oder mechanisch/technologisch schwächeren Systemteiles. Damit ist auch der erfindungsgemässe Anspruch unterlegt, wonach eine nach der Erfindung ausgestaltete Endoprothese (Bipaarungsprothese) als System eine höhere Festigkeit (Bruchfestigkeit) aufweist, als. die Bruchfestigkeit ihres schwächsten Teiles normalerweise erwarten lässt.

Die vergleichenden Versuche stellen eine Monopaarungsprothese (Al₂O₃/Al₂O₃) einer Bipaarungsprothese (ZrO₂/Al₂O₃) gegenüber. Ersatz von Al₂O₃ der Bipaarungsprothese durch die übrigen genannten Werkstoffe ergibt bezüglich der Verschleissfestigkeit des ZrO₂ Teiles der Bipaarungsprothese durch Messung und Vergleich gleiche oder zumindest gleichwertige Ergebnisse, wenn der Werkstoff mit höherer Wärmeleitfähgikeit eine solche besitzt, die um den Faktor 4 bis 200 vorzugsweise 8 bis 100 höher ist als die des Werkstoffes mit niedriger Wärmeleitfähigkeit (Zirkonoxid). Die Belastbarkeiten sind unterschiedlich, dies ist für den erfindungsgemässen Erfolg jedoch unerheblich, da der aus dem ZrO₂/Al₂O₃ abgeleitete Belastbarkeitsgrundsatz für alle übrigen beanspruchten Materialpaarungen seine Gültigkeit behält.

## Patentansprüche

1. Ein endoprothetisches Gelenk aus keramischem Werkstoff umfassend einen ersten Gelenkteil aus Zirkonoxid und einen zweiten Gelenkteil **dadurch gekennzeichnet, dass** der zweite Gelenkteil aus einem Werkstoff gefertigt ist, ausgewahlt aus einer Gruppe bestehend aus Aluminiumoxid, Aluminiumnitrid, Bornitrid, Berylliumoxid, Kohlenstoff und Siliziumkarbid, wobei der Werkstoff des zweiten Gelenkteiles eine Wärmeleitfähigkeit aufweist, die die Wärmeleitfähigkeit des Zirkonoxides um einen Faktor zwischen vier bis zweihundert übersteigt.

2. Gelenk gemäss Anspruch 1, **dadurch gekennzeichnet, dass** der Faktor zwischen acht und einhundert liegt.

3. Gelenk gemäss Anspruch 1, **dadurch gekennzeichnet, dass** der Werkstoff des zweiten Gelenkteiles eine Wärmeleitfähigkeit zwischen zehn und dreihundert pro Meter Kelvin (W/mK) aufweist.

4. Gelenk gemäss Anspruch 1, **dadurch gekennzeichnet, dass** der Werkstoff des zweiten Gelenkteiles eine Wärmeleitfähigkeit zwischen zwanzig und zweihundert W/mK aufweist.

5. Gelenk nach Anspruch 1, **dadurch gekennzeichnet, dass** der erste Gelenkteil eine Kugel und der zweite Gelenkteil eine Pfanne zur Aufnahme der Kugel oder umgekehrt ist.

## Claims

1. An endoprothetic joint of ceramic material comprising a first joint portion of zirconium oxide and a second joint portion, **characterised** thereby that the second joint portion is formed of a material selected from a group comprising aluminium oxide, aluminium nitride, boron nitride, beryllium oxide, carbon and silicon carbide, whereby the material of the second joint portion has a thermal conductivity which exceeds the thermal conductivity of the zirconium oxide by a factor between four up to two hundred.

2. Joint according to claim 1, **characterised that** the factor lies between eight and one hundred .

3. Joint according to claim 1, **characterised** thereby that the material of the second joint portion has a thermal conductivity between ten and three hundred watts per meter Kelvin (W/mK).

4. Joint according to claim 1, **characterised that** the material of the second joint portion has a thermal conductivity between twenty and two hundred watts per meter Kelvin (W/mK).

5. Joint according to claim 1, **characterised** thereby that the first joint portion is a ball and the second joint portion is a socket for receiving the ball or vice-versa.

## Revendications

1. Articulation endoprothétique en matière céramique, comprenant un premier élément d'articulation en oxyde de zirconium et un deuxième élément d'articulation, **caractérisée en ce que** le deuxième élément d'articulation est réalisé dans une matière choisie dans un groupe constitué de l'oxyde d'aluminium, du nitrure d'aluminium, du nitrure de bore, de l'oxyde de béryllium, du carbone et du carbure de silicium, moyennant quoi la matière du deuxième élément d'articulation présente une conductivité thermique qui dépasse la conductivité thermique de l'oxyde de zirconium d'un facteur compris entre quatre et deux cents.

2. Articulation selon la revendication 1, **caractérisée en ce que** le facteur est compris entre huit et cent.

3. Articulation selon la revendication 1, **caractérisée en ce que** la matière du deuxième élément d'articulation présente une conductivité thermique comprise entre dix et trois cents par mètre kelvin (W/mK).

4. Articulation selon la revendication 1, **caractérisée en ce que** la matière de la deuxième articulation présente une conductivité comprise entre vingt et deux cents W/mK.

5. Articulation selon la revendication 1, **caractérisée en ce que** le premier élément d'articulation est une boule et **en ce que** le deuxième élément d'articulation est une cavité permettant de recevoir la boule ou inversement.
